# EUROPÄISCHE PATENTSCHRIFT

(11) **EP 1 106 600 B1**
(45) Veröffentlichungstag und Bekanntmachung des Hinweises auf die Patenterteilung: **04.08.2004**
(21) Anmeldenummer: 00124609.9
(22) Anmeldetag: 10.11.2000
(51) Int. Cl.: C07C 209/26

(54) **Verfahren zur Herstellung von Aminen**
Process for the preparation of amines
Procédé pour la préparation d'amines

(30) Priorität: 06.12.1999 DE 19958700
(43) Veröffentlichungstag der Anmeldung: 13.06.2001
(73) Patentinhaber: BASF AKTIENGESELLSCHAFT, 67056 Ludwigshafen (DE)
(72) Erfinder: Nouwen, Jan, Dr., 64653 Lorsch (DE); Höhn, Arthur, Dr., 67281 Kirchheim (DE); Neuhauser, Horst, Dr., 37373 Dudenhofen (DE); Funke, Frank, Dr., 67227 Frankenthal (DE); Schunk, Stephan Andreas, 69115 Heidelberg (DE); Melder, Johann-Peter, Dr., 67459 Böhl-Iggelheim (DE); Eger, Knut, Dr., 67117 Limburgerhof (DE); Hesse, Michael, Dr., 67549 Worms (DE); Wulff-Döring, Joachim, Dr., 67105 Schifferstadt (DE)

(56) Entgegenhaltungen:
- EP-A- 0 382 049
- EP-A- 0 514 692

## Beschreibung

Die vorliegende Erfindung betrifft ein Verfahren zur Herstellung von Aminen durch Umsetzung von Aldehyden oder Ketonen bei erhöhter Temperatur und erhöhtem Druck mit Stickstoffverbindungen, ausgewählt aus der Gruppe Ammoniak, primäre und sekundäre Amine, und mit Wasserstoff in Gegenwart eines Katalysators.

Aus der EP-A-514 692 sind Kupfer, Nickel und/oder Kobalt, Zirkonium- und/oder Aluminiumoxid enthaltende Katalysatoren zur katalytischen Aminierung von Alkoholen in der Gasphase mit Ammoniak oder primären Aminen und Wasserstoff bekannt.

Diese Patentanmeldung lehrt, dass bei diesen Katalysatoren das Atomverhältnis von Nickel zu Kupfer 0,1 bis 1,0, bevorzugt 0,2 bis 0,5 (Seite 2, Zeilen 47 bis 48; vergl. auch loc. cit.: Beispiel 1), betragen muss, da ansonsten bei der Aminierung von Alkoholen in erhöhtem Maße ausbeutemindernde Nebenprodukte auftreten (loc. cit.: Beispiele 6 und 12). Als Träger wird bevorzugt Aluminiumoxid (loc. cit.: Beispiele 1 bis 5 und 7 bis 11) verwendet.

Aus der EP-A-382 049 sind Katalysatoren, die sauerstoffhaltige Zirkonium-, Kupfer-, Kobalt- und Nickelverbindungen enthalten, und Verfahren zur hydrierenden Aminierung von Alkoholen oder Carbonylverbindungen bekannt. Der bevorzugte Zirkoniumoxidgehalt dieser Katalysatoren beträgt 70 bis 80 Gew.-% (loc. cit.: Seite 2, letzter Absatz; Seite 3, 3. Absatz; Beispiele). Diese Katalysatoren zeichnen sich zwar durch eine gute Aktivität und Selektivität aus, besitzen allerdings verbesserungswürdige Standzeiten.

Aus der EP-A-696 572 und der EP-A-697 395 sind Nickel-, Kupfer-, Zirkonium- und Molybdänoxid enthaltende Katalysatoren zur katalytischen Aminierung von Alkoholen mit Stickstoffverbindungen in Gegenwart von Wasserstoff bekannt.

Aus der älteren deutschen Anmeldung Nr. 19910960.5 vom 12.03.99 sind Nickel-, Kupfer- und Zirkoniumoxid enthaltende Katalysatoren zur katalytischen Aminierung von Aldehyden oder Ketonen mit Stickstoffverbindungen in Gegenwart von Wasserstoff bekannt. Die Katalysatoren enthalten bevorzugt keine katalytisch aktiven Mengen an Kobalt. Diese Katalysatoren zeichnen sich zwar durch eine gute Aktivität aus, besitzen allerdings verbesserungswürdige mechanische Stabilitäten und Selektivitäten.

Die EP-A-905 122 betrifft ein Verfahren zur Herstellung von Aminen durch Umsetzung von primären oder sekundären Alkoholen mit Stickstoffverbindungen, ausgewählt aus der Gruppe Ammoniak, primäre und sekundäre Amine, bei erhöhten Temperaturen und Drücken mit Wasserstoff in Gegenwart von Katalysatoren enthaltend Zirkonium, Kupfer und Nickel und kein Kobalt.

Die ältere europäische Anmeldung Nr. 99111282.2 vom 10.06.99 betrifft ein Verfahren zur Herstellung von Aminen durch Umsetzung von primären oder sekundären Alkoholen mit Stickstoffverbindungen, ausgewählt aus der Gruppe Ammoniak, primäre und sekundäre Amine, bei erhöhten Temperaturen und Drücken mit Wasserstoff in Gegenwart von Katalysatoren enthaltend Zirkonium, Kupfer, Nickel und Kobalt.

Die ältere deutsche Anmeldung Nr. 19859776.2 vom 23.12.98 beschreibt ein Verfahren zur Herstellung von Aminen durch Umsetzung von primären oder sekundären Alkoholen, Aldehyden oder Ketonen mit Stickstoffverbindungen bei erhöhten Temperaturen und Drücken in Gegenwart von Wasserstoff und eines Katalysators enthaltend Kupfer und sauerstoffhaltige Verbindungen des Titans, wobei man den Katalysator in Form von Formkörpern einsetzt, die unter Zusatz von metallischem Kupferpulver hergestellt wurden.

DE-A-28 38 184 beschreibt ein Verfahren zur Herstellung tertiärer Amine durch Umsetzung sekundärer Amine mit Alkoholen oder Carbonylverbindungen unter hydrierenden Bedingungen in der Gasphase, indem man die Umsetzung an einem Kupferkatalysator vornimmt, der durch thermische Zersetzung und Reduktion eines basischen Kupfer-Aluminium-Carbonats erhalten worden ist.

Nachteilig an den Verfahren des Stands der Technik ist, dass bei der aminierenden Hydrierung von Aldehyden oder Ketonen zu geringe Selektivitäten und Ausbeuten erreicht werden und/oder die Katalysatoren eine ungenügende Aktivität und/oder Stabilität unter den Reaktionsbedingungen aufweisen.

Der vorliegenden Erfindung lag die Aufgabe zugrunde, unter Abhilfe der Nachteile des Stands der Technik die Wirtschaftlichkeit bisheriger Verfahren zur hydrierenden Aminierung von Aldehyden und Ketonen zu verbessern. Es sollten Katalysatoren gefunden werden, die technisch in einfacher Weise herzustellen sind und die es erlauben, die hydrierende Aminierung von Aldehyden und Ketonen mit hohem Aldehyd- bzw. Keton-Umsatz, insbesondere Aldehyd- bzw.

Keton-Umsätzen von 90 bis 100 %, hoher Ausbeute, hoher Selektivität, insbesondere Selektivitäten von 95 bis 100 % (bezogen auf den Aldehyd bzw. das Keton), und hoher Katalysatorstandzeit bei gleichzeitig hoher mechanischer Stabilität des Katalysatorformkörpers (z. B. gemessen als Seitendruckfestigkeit) durchzuführen. Die Katalysatoren sollen demnach eine hohe Aktivität und unter den Reaktionsbedingungen eine hohe chemische und mechanische Stabilität aufweisen.

Demgemäß wurde ein Verfahren zur Herstellung von Aminen durch Umsetzung von Aldehyden oder Ketonen bei Temperaturen von 50 bis 250°C und Drücken von 0,1 bis 40 MPa mit Stickstoffverbindungen, ausgewählt aus der Gruppe Ammoniak, primäre und sekundäre Amine, und mit Wasserstoff in Gegenwart eines Katalysators gefunden, welches dadurch gekennzeichnet ist, dass die katalytisch aktive Masse des Katalysators nach dessen Herstellung und vor der Behandlung mit Wasserstoff 22 bis 45 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
5 bis 50 Gew.-% sauerstoffhaltige Verbindungen des Nickels, berechnet als NiO, wobei das Molverhältnis von Nickel zu Kupfer größer 1 ist,
5 bis 50 Gew.-% sauerstoffhaltige Verbindungen des Kobalts, berechnet als CoO,
0 bis 5 Gew.-% sauerstoffhaltige Verbindungen des Molybdäns, berechnet als MoO₃,
und 0 bis 10 Gew.-% sauerstoffhaltige Verbindungen des Aluminiums und/oder Mangans, berechnet als Al₂O₃ bzw. MnO₂, enthält.

Im allgemeinen werden im erfindungsgemäßen Verfahren die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch inaktiven Begleitstoffe enthalten.

Die katalytisch aktive Masse kann nach Mahlung als Pulver oder als Splitt in das Reaktionsgefäß eingebracht oder bevorzugt, nach Mahlung, Vermischung mit Formhilfsmitteln, Formung und Temperung, als Katalysatorformkörper - beispielsweise als Tabletten, Kugeln, Ringe, Extrudate (z. B. Stränge) - in den Reaktor eingebracht werden.

Die Konzentrationsangaben (in Gew.-%) der Komponenten des Katalysators beziehen sich jeweils - falls nicht anders angegeben - auf die katalytisch aktive Masse des hergestellten Katalysators nach dessen letzter Wärmebehandlung und vor der Behandlung mit Wasserstoff.

Die katalytisch aktive Masse des Katalysators, nach dessen letzter Wärmebehandlung und vor der Behandlung mit Wasserstoff, ist als die Summe der Massen der katalytisch aktiven Bestandteile und der Trägermaterialien definiert und enthält im wesentlichen sauerstoffhaltige Verbindungen des Zirkoniums, sauerstoffhaltige Verbindungen des Kupfers, sauerstoffhaltige Verbindungen des Nikkels, sauerstoffhaltige Verbindungen des Kobalts sowie optional sauerstoffhaltige Verbindungen des Molybdäns und/oder sauerstoffhaltige Verbindungen des Aluminiums und/oder sauerstoffhaltige Verbindungen des Mangans.

Die Summe der o.g. katalytisch aktiven Bestandteile und der o.g. Trägermaterialien in der katalytisch aktiven Masse, berechnet als ZrO₂, CuO, NiO, CoO, MoO₃, Al₂O₃ und MnO₂, beträgt üblicherweise 70 bis 100 Gew.-%, bevorzugt 80 bis 100 Gew.-%, besonders bevorzugt 90 bis 100 Gew.-%, insbesondere 95 bis 100 Gew.-%, ganz besonders 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:
Übergangsmetalle, wie Re bzw. Rheniumoxide, Cr bzw. Chromoxide, W bzw. Wolframoxide, Ta bzw. Tantaloxide, Nb bzw. Nioboxide oder Nioboxalat, V bzw. Vanadiumoxide bzw. Vanadylpyrophosphat, Lanthanide, wie Ce bzw. CeO₂ oder Pr bzw. Pr₂O₃, Alkalimetalloxide, wie Na₂O, Alkalimetallcarbonate, wie Na₂CO₃ und K₂CO₃, Erdalkalimetalloxide, wie SrO, Erdalkalimetallcarbonate, wie MgCO₃, CaCO₃ und BaCO₃, Boroxid (B₂O₃).
Die katalytisch aktive Masse der im erfindungsgemäßen Verfahren eingesetzten Katalysatoren enthält nach dessen Herstellung und vor der Behandlung mit Wasserstoff
22 bis 45 Gew.-%, bevorzugt 22 bis 39 Gew.-%, besonders bevorzugt 25 bis 39 Gew.-%, sauerstoffhaltige Verbindungen des Zirkoniums, berechnet als ZrO₂,
1 bis 30 Gew.-%, bevorzugt 2 bis 25 Gew.-%, besonders bevorzugt 5 bis 15 Gew.-%, sauerstoffhaltige Verbindungen des Kupfers, berechnet als CuO,
5 bis 50 Gew.-%, bevorzugt 15 bis 45 Gew.-%, besonders bevorzugt 21 bis 40 Gew.-%, sauerstoffhaltige Verbindungen des Nickels, berechnet als NiO, wobei das Molverhältnis Nickel zu Kupfer größer 1, bevorzugt größer 1,2, besonders bevorzugt 1,5 bis 8,5, ist,
5 bis 50 Gew.-%, bevorzugt 20 bis 45 Gew.-%, besonders bevorzugt 21 bis 40 Gew.-%, sauerstoffhaltige Verbindungen des Kobalts, berechnet als CoO,
0 bis 5 Gew.-%, besonders bevorzugt 0,1 bis 0,5 Gew.-%, sauerstoffhaltige Verbindungen des Molybdäns, berechnet als MoO₃, und
0 bis 10 Gew.-% sauerstoffhaltige Verbindungen des Aluminiums und/oder Mangans, berechnet als Al₂O₃ bzw. MnO₂, wobei das Gewichtsverhältnis der sauerstoffhaltigen Verbindungen des Zirkoniums, berechnet als ZrO₂, zu den sauerstoffhaltigen Verbindungen des Aluminiums und/oder Mangans, berechnet als Al₂O₃ bzw. MnO₂, bevorzugt mindestens 2,5, besonderes bevorzugt mindestens 5, beträgt, ganz besonderes bevorzugt 0 Gew.-% sauerstoffhaltige Verbindungen des Aluminiums und/oder Mangans.

Bevorzugt werden im erfindungsgemäßen Verfahren Katalysatoren eingesetzt, deren katalytisch aktive Masse nach dessen Herstellung und vor der Behandlung mit Wasserstoff 5 bis 15 Gew.-% sauerstoffhaltige Verbindungen des Kupfers, berechnet als CuO, und in Summe 35 bis 69 Gew.-% sauerstoffhaltige Verbindungen des Nikkels, berechnet als NiO, und sauerstoffhaltige Verbindungen des Kobalts, berechnet als CoO, enthält, wobei der Gehalt an sauerstoffhaltigen Verbindungen des Kobalts, berechnet als CoO, bezogen auf die Summe an sauerstoffhaltigen Verbindungen des Nickels, berechnet als NiO, und sauerstoffhaltigen Verbindungen des Kobalts, berechnet als CoO, mindestens 1,7 Gew.-%, insbesondere mindestens 12,0 Gew.-%, ganz besonders mindestens 40 Gew.-%, beträgt.

Zur Herstellung der Katalysatoren sind verschiedenerlei Verfahrensweisen möglich. Sie sind beispielsweise durch Peptisieren pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Im allgemeinen werden zur Herstellung der erfindungsgemäßen Katalysatoren jedoch Fällungsmethoden angewandt. So können sie beispielsweise durch eine gemeinsame Fällung der Nickel- Kobalt- und Kupferkomponenten aus einer diese Elemente enthaltenden, wässrigen Salzlösung mittels Mineralbasen in Gegenwart einer Aufschlämmung einer schwerlöslichen, sauerstoffhaltigen Zirkoniumverbindung und anschließendes Waschen, Trocknen und Calcinieren des erhaltenen Präzipitats erhalten werden. Als schwerlösliche, sauerstoffhaltige Zirkoniumverbindungen können beispielsweise Zirkoniumdioxid, Zirkoniumoxidhydrat, Zirkoniumphosphate, -borate und -silikate Verwendung finden. Die Aufschlämmungen der schwerlöslichen Zirkoniumverbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Zirkoniumverbindungen aus wässrigen Zirkoniumsalzlösungen mittels Mineralbasen erhalten.

Insbesondere werden die erfindungsgemäßen Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200 °C, vorzugsweise bei 100 bis 150 °C, getrocknet und danach calciniert. Die Calcinierung (Wärmebehandlung) wird im allgemeinen bei Temperaturen zwischen 300 und 800 °C, vorzugsweise bei 400 bis 600 °C, insbesondere bei 450 bis 550 °C ausgeführt.

Nach der Calcinierung wird der Katalysator zweckmäßigerweise konditioniert, sei es, dass man ihn durch Vermahlen auf eine bestimmte Korngröße einstellt oder dass man ihn nach seiner Vermahlung mit Formhilfsmitteln wie Graphit oder Stearinsäure vermischt, mittels einer Tablettenpresse zu Formlingen verpreßt und tempert (wärmebehandelt). Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die hergestellten Katalysatoren können als solche gelagert werden. Vor ihrem Einsatz als Katalysatoren zur hydrierenden Aminierung von Aldehyden oder Ketonen werden sie üblicherweise durch Behandlung mit Wasserstoff vorreduziert. Sie können jedoch auch ohne diese Vorreduktion eingesetzt werden, wobei sie dann unter den Bedingungen der hydrierenden Aminierung durch den im Reaktor vorhandenen Wasserstoff reduziert werden. Zur Vorreduktion werden die Katalysatoren im allgemeinen zunächst bei 150 bis 200 °C über einen Zeitraum von 12 bis 20 Stunden einer Stickstoff-Wasserstoff-Atmosphäre ausgesetzt und anschließend noch bis zu ca. 24 Stunden bei 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Ein besonderer Vorteil der im erfindungsgemäßen Verfahren eingesetzten Katalysatoren ist deren mechanische Stabilität, d.h. deren Härte. Die mechanische Stabilität kann durch die Messung der sogenannten Seitendruckfestigkeit bestimmt werden. Hierzu wird der Katalysatorformkörper, z. B. die Katalysatortablette, zwischen zwei parallelen Platten mit zunehmender Kraft belastet, wobei diese Belastung z. B. auf der Mantelseite von Katalysatortabletten erfolgen kann, bis ein Bruch des Katalysatorformkörpers eintritt. Die beim Bruch des Katalysatorformkörpers registrierte Kraft ist die Seitendruckfestigkeit.

### Amine der allgemeinen Formel I

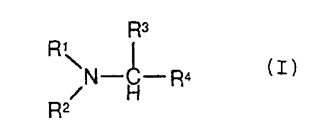

in der
R¹, R²
Wasserstoff, C₁₋₂₀-Alkyl, C₃₋₁₂-Cycloalkyl, Aryl, C₇₋₂₀-Aralkyl und
C₇₋₂₀-Alkylaryl oder gemeinsam (CH₂)ⱼ-X-(CH₂)ₖ,
R³, R⁴
Wasserstoff, Alkyl, wie C₁₋₂₀₀-Alkyl, Cycloalkyl, wie C₃₋₁₂-Cycloalkyl, Hydroxyalkyl, wie C₁₋₂₀-Hydroxyalkyl, Aminoalkyl, wie C₁₋₂₀-Aminoalkyl, Hydroxyalkylaminoalkyl, wie C₁₋₂₀- Hydroxyalkylaminoalkyl, Alkoxyalkyl, wie C₂₋₃₀-Alkoxyalkyl, Dialkylaminoalkyl, wie C₃₋₃₀-Dialkylaminoalkyl, Alkylaminoalkyl, wie C₂₋₃₀-Alkylaminoalkyl, R⁵-(OCR⁶R⁷CR⁸R⁹)ₙ-(OCR⁶R⁷), Aryl, Heteroaryl, Aralkyl, wie C₇₋₂₀-Aralkyl, Heteroarylalkyl, wie C₄₋₂₀-Heteroarylalkyl, Alkylaryl, wie C₇₋₂₀-Alkylaryl, Alkylheteroaryl, wie C₄₋₂₀-Alkylheteroaryl und Y-(CH₂)ₘ-NR⁵-(CH₂)_{q} oder gemeinsam (CH₂)₁-X-(CH₂)ₘ oder
R² und R⁴
gemeinsam (CH₂)ₗ-X-(CH₂)ₘ,
R⁵, R¹⁰
Wasserstoff, C₁₋₄-Alkyl, C₇₋₄₀-Alkylphenyl,
R⁶, R⁷, R⁸, R⁹
Wasserstoff, Methyl oder Ethyl,
X
CH₂, CHR⁵, Sauerstoff (O), Schwefel (S) oder NR⁵,
Y
N(R¹⁰)₂, Hydroxy, C₂₋₂₀-Alkylaminoalkyl oder C₃₋₂₀-Dialkylaminoalkyl,
n
eine ganze Zahl von 1 bis 30 und
j, k, l, m, q
eine ganze Zahl von 1 bis 4,
bedeuten, sind wirtschaftlich von besonderem Interesse.

Das erfindungsgemäße Verfahren findet daher bevorzugt zur Herstellung der Amine I Anwendung, indem man Aldehyde oder Ketone der Formel II bzw. III 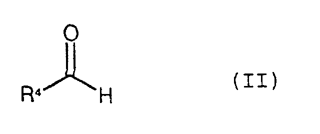 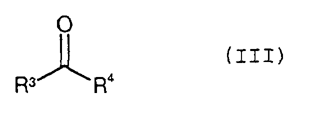 mit Stickstoffverbindungen der allgemeinen Formel IV 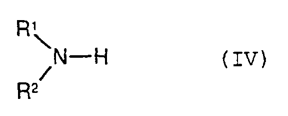 wobei R¹, R², R³ und R⁴ die oben genannten Bedeutungen haben, umsetzt.

Wie aus den Definitionen für die Reste R² und R⁴ hervorgeht, kann die Umsetzung auch intramolekular in einem entsprechenden Aminoketon oder Aminoaldehyd erfolgen.

Die Substituenten R¹, R², R³, R⁴, R⁵, R⁶, R⁷, R⁸, R⁹, R¹⁰, die Variablen X, Y, und die Indizes j, k, l, m, n und q in den Verbindungen I, II, III und IV haben unabhängig voneinander folgende Bedeutungen:
R¹, R², R³, R⁴, R⁵, R⁶, R⁷, R⁸, R⁹, R¹⁰
   - Wasserstoff (H),
R³, R⁴
   - C₁₋₂₀₀-Alkyl, bevorzugt C₁₋₁₄-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, Cyclohexylmethyl, n-Octyl, iso-Octyl, 2-Ethylhexyl, n-Decyl, 2-n-Propyl-n-heptyl, n-Tridecyl, 2-n-Butyl-n-nonyl und 3-n-Butyl-n-nonyl, besonders bevorzugt iso-Propyl, 2-Ethylhexyl, n-Decyl, 2-n-Propyl-n-heptyl, n-Tridecyl, 2-n-Butyl-n-nonyl und 3-n-Butyl-n-nonyl sowie bevorzugt C₄₀₋₂₀₀-Alkyl, wie Polybutyl, Polyisobutyl, Polypropyl, Polyisopropyl und Polyethyl, besonders bevorzugt Polybutyl und Polyisobutyl,
   - C₁₋₂₀-Hydroxyalkyl, bevorzugt C₁₋₈-Hydroxyalkyl, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methyl-ethyl,
   - C₁₋₂₀-Aminoalkyl, bevorzugt C₁₋₈-Aminoalkyl, wie Aminomethyl, 2-Aminoethyl, 2-Amino-1,1-dimethylethyl, 2-Amino-n-propyl, 3-Amino-n-propyl, 4-Amino-n-butyl, 5-Amino-n-pentyl, N-(Aminoethyl)aminoethyl und N-(Aminoethyl)aminomethyl,
   - C₁₋₂₀-Hydroxyalkylaminoalkyl, bevorzugt C₁₋₈-Hydroxyalkylaminoalkyl, wie (2-Hydroxyethylamino)methyl, 2-(2-Hydroxyethylamino)ethyl und 3-(2-Hydroxyethylamino)propyl,
   - C₂₋₃₀-Alkoxyalkyl, bevorzugt C₂₋₂₀-Alkoxyalkyl, besonders bevorzugt C₂₋₈₋Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl, besonders bevorzugt C₂- bis C₄-Alkoxyalkyl, wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl,
   - R⁵-(OCR⁶R⁷CR⁸R⁹)ₙ-(OCR⁶R⁷), bevorzugt R⁵-(OCHR⁷CHR⁹)ₙ-(OCR⁶R⁷), besonders bevorzugt R⁵-(OCH₂CHR⁹)ₙ-(OCR⁶R⁷),
   - C₃₋₃₀-Dialkylaminoalkyl, bevorzugt C₃₋₂₀-Dialkylaminoalkyl, besonders bevorzugt C₃₋₁₀- N,N-Dialkylaminoalkyl, wie N,N-Dimethylaminomethyl, 2-(N,N-Dibutylamino)methyl, 2-(N,N-Dimethylamino)ethyl, 2-(N,N-Diethylamino)ethyl, 2-(N,N-Dibutylamino)ethyl, 2-(N,N-Di-n-propylamino)ethyl und 2-(N,N-Di-isopropylamino) ethyl, (R⁵)₂N-(CH₂)_{q},
   - C₂₋₃₀-Alkylaminoalkyl, bevorzugt C₂₋₂₀-Alkylaminoalkyl, besonders bevorzugt C₂₋₈-Alkylaminoalkyl, wie Methylaminomethyl, Methylaminoethyl, Ethylaminomethyl, Ethylaminoethyl und iso-Propylaminoethyl, (R⁵)HN-(CH₂)_{q},
   - Y-(CH₂)ₘ-NR⁵-(CH₂)_{q},
   - C₄₋₂₀-Heteroarylalkyl, wie Pyrid-2-yl-methyl, Furan-2-yl-methyl, Pyrrol-3-yl-methyl und Imidazol-2-yl-methyl,
   - C₄₋₂₀-Alkylheteroaryl, wie 2-Methyl-3-pyridinyl, 4,5-Dimethylimidazol-2-yl, 3-Methyl-2-furanyl und 5-Methyl-2-pyrazinyl,
   - Heteroaryl, wie 2-Pyridinyl, 3-Pyridinyl, 4-Pyridinyl, Pyrazinyl, Pyrrol-3-yl, Imidazol-2-yl, 2-Furanyl und 3-Furanyl,
R¹_{,} R², R³, R⁴
   - C₃₋₁₂-Cycloalkyl, bevorzugt C₃₋₈-Cycloalkyl, wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
   - Aryl, wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - C₇₋₂₀-Alkylaryl, bevorzugt C₇₋₁₂-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
   - C₇₋₂₀-Aralkyl, bevorzugt C₇₋₁₂-Phenylalkyl, wie Benzyl, p-Methoxybenzyl, 3,4-Dimethoxybenzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
   - R³ und R⁴ oder R² und R⁴ gemeinsam eine -(CH₂)ₗ-X-(CH₂)ₘ-Gruppe, , wie -(CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)₂-O-(CH₂)₂-, -(CH₂)₂-NR⁵-(CH₂)₂-, -CH₂-O-(CH₂)₃-, -CH₂-NR⁵-(CH₂)₃-,
R¹, R²
   - C₁₋₂₀-Alkyl, bevorzugt C₁₋₈-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁₋₄-Alkyl wie Methyl, Ethyl, n-Propyl, iso-Propyl, n-Butyl, iso-Butyl, sec-Butyl und tert.-Butyl,
   - R¹ und R² gemeinsam eine -(CH₂)ⱼ-X-(CH₂)ₖ- Gruppe, wie -(CH₂)₃-, -(CH₂)₄-, -(CH₂)₅-, -(CH₂)₆-, -(CH₂)₇-, -(CH₂)-O-(CH₂)₂-, -(CH₂)-NR⁵-(CH₂)₂-, -(CH₂)₂-O-(CH₂)₂-, -(CH₂)₂-NR⁵-(CH₂)₂-, -CH₂-O-(CH₂)₃-, -CH₂-NR⁵-(CH₂)₃-,
R⁵, R¹⁰
   - C₁₋₄-Alkyl, wie Methyl, Ethyl, n-Propyl, iso-Propyl, n-Butyl, iso-Butyl, sec.-Butyl und tert.-Butyl, bevorzugt Methyl und Ethyl, besonders bevorzugt Methyl,
   - C₇₋₄₀-Alkylphenyl, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2-, 3-, 4-Nonylphenyl, 2-, 3-, 4-Decylphenyl, 2,3-, 2,4-, 2,5-, 3,4-, 3,5-Dinonylphenyl, 2,3-, 2,4-, 2,5-, 3,4- und 3,5-Didecylphenyl,
R⁶, R⁷, R⁸, R⁹
   - Methyl oder Ethyl, bevorzugt Methyl,
X
   - CH₂, CHR⁵, Sauerstoff (O), Schwefel (S) oder NR⁵, bevorzugt CH₂ und O,
Y
   - N(R¹⁰)₂, bevorzugt NH₂ und N(CH₃)₂,
   - Hydroxy,
   - C₂₋₂₀-Alkylaminoalkyl, bevorzugt C₂₋₁₆-Alkylaminoalkyl, wie Methylaminomethyl, Methylaminoethyl, Ethylaminomethyl, Ethylaminoethyl und iso-Propylaminoethyl,
   - C₃₋₂₀-Dialkylaminoalkyl, bevorzugt C₃₋₁₆-Dialkylaminoalkyl, wie Dimethylaminomethyl, Dimethylaminoethyl, Diethylaminoethyl, Di-n-propylaminoethyl und Di-iso-propylaminoethyl, j, l
   - eine ganze Zahl von 1 bis 4 wie 1, 2, 3 und 4, bevorzugt 2 und 3, besonders bevorzugt 2,
k, m, q
   - eine ganze Zahl von 1 bis 4 wie 1, 2, 3 und 4, bevorzugt 2, 3 und 4, besonders bevorzugt 2 und 3,
n
   - eine ganze Zahl von 1 bis 10, bevorzugt eine ganze Zahl von 1 bis 8 wie 1, 2, 3, 4, 5, 6, 7 oder 8, besonders bevorzugt eine ganze Zahl von 1 bis 6 wie 1, 2, 3, 4, 5 oder 6.

Als im erfindungsgemäßen Verfahren einsetzbare Ketone eignen sich praktisch alle aliphatischen und aromatischen Ketone. Die aliphatischen Ketone können geradkettig, verzweigt oder zyklisch sein, die Ketone können Heteroatome enthalten. Hinsichtlich der Kohlenstoffzahl der aminierbaren Ketone sind bislang keine Beschränkungen bekannt. Die Ketone können ferner Substituenten tragen, welche sich unter den Bedingungen der hydrierenden Aminierung inert verhalten, beispielsweise Alkoxy-, Alkenyloxy-, Alkylamino- oder Dialkylaminogruppen. Sollen mehrwertige Ketone aminiert werden, so hat man es über die Steuerung der Reaktionsbedingungen in der Hand, Aminoketone, Aminoalkohole, zyklische Amine oder mehrfach aminierte Produkte zu erhalten.

Bevorzugt werden beispielsweise die folgenden Ketone aminierend hydriert:

Aceton, Ethylmethylketon, Methylvinylketon, Isobutylmethylketon, 3-Methylbutan-2-on, Diethylketon, Tetralon, Acetophenon, p-Methyl-acetophenon, p-Methoxy-acetophenon, m-Methoxy-acetophenon, 1-Acetyl-naphthalin, 2-Acetyl-naphthalin, 1-Phenyl-3-butanon, Cyclobutanon, Cyclopentanon, Cyclopentenon, Cyclohexanon, Cyclohexenon, 2,6-Dimethylcyclohexanon, Cycloheptanon, Cyclododecanon, Acetylaceton, Methylglyoxal und Benzophenon.

Als im erfindungsgemäßen Verfahren einsetzbare Aldehyde eignen sich praktisch alle aliphatischen und aromatischen Aldehyde. Die aliphatischen Aldehyde können geradkettig, verzweigt oder zyklisch sein, die Aldehyde können Heteroatome enthalten. Hinsichtlich der Kohlenstoffzahl der aminierbaren Aldehyde sind bislang keine Beschränkungen bekannt. Die Aldehyde können ferner Substituenten tragen, welche sich unter den Bedingungen der hydrierenden Aminierung inert verhalten, beispielsweise Alkoxy-, Alkenyloxy-, Alkylamino- oder Dialkylaminogruppen. Sollen mehrwertige Aldehyde oder Ketoaldehyde aminiert werden, so hat man es über die Steuerung der Reaktionsbedingungen in der Hand, Aminoalkohole, cyclische Amine oder mehrfach aminierte Produkte zu erhalten.

Bevorzugt werden beispielsweise die folgenden Aldehyde aminierend hydriert:

Formaldehyd, Acetaldehyd, Propionaldehyd, n-Butyraldehyd, Isobutyraldehyd, Pivalinaldehyd, n-Pentanal, n-Hexanal, 2-Ethylhexanal, 2-Methylpentanal, 3-Methylpentanal, 4-Methylpentanal, Glyoxal, Benzaldehyd, p-Methoxybenzaldehyd, p-Methylbenzaldehyd, Phenylacetaldehyd, (p-Methoxy-phenyl)acetaldehyd, (3,4-Dimethoxyphenyl)acetaldehyd, 4-Formyltetrahydropyran, 3- Formyltetrahydrofuran, 5-Formylvaleronitril, Citronellal, Acrolein, Methacrolein, Ethylacrolein, Citral, Crotonaldehyd, 3-Methoxypropionaldehyd, 3-Aminopropionaldehyd, Hydroxypivalinaldehyd, Dimethylolpropionaldehyd, Dimethylolbutyraldehyd, Furfural, Glyoxal, Glutaraldehyd sowie hydroformylierte Oligomere und Polymere, wie z. B. hydroformyliertes Polyisobuten (Polyisobutenaldehyd) oder durch Metathese von 1-Penten und Cyclopenten erhaltenes und hydroformyliertes Oligomer.

Als Aminierungsmittel bei der hydrierenden Aminierung von Aldehyden und Ketonen können sowohl Ammoniak als auch primäre oder sekundäre, aliphatische, cycloaliphatische oder aromatische Amine eingesetzt werden.

Bei Verwendung von Ammoniak als Aminierungsmittel werden die Carbonylgruppen zunächst in freie Aminogruppen (-NH₂) umgewandelt. Die so gebildeten primären Amine können mit Hydroxyl- oder weiteren Carbonylgruppen zu den entsprechenden sekundären Aminen und diese wiederum mit Hydroxyl- oder weiteren Carbonylgruppen zu den entsprechenden, gegebenenfalls symmetrischen, tertiären Aminen reagieren. Je nach Zusammensetzung des Reaktionsansatzes und je nach den angewandten Reaktionsbedingungen - Druck, Temperatur, Reaktionszeit, Molverhältnisse - lassen sich auf diese Weise je nach Wunsch bevorzugt primäre, sekundäre oder tertiäre Amine darstellen.

Aus Aldehyden oder Ketonen mit mehr als einer Aldehyd- oder Ketogruppe oder aus Ketoaldehyden lassen sich auf diese Weise durch intramolekulare hydrierende Aminierung zyklische Amine wie Pyrrolidine, Piperidine, Hexamethylenamine, Piperazine und Morpholine herstellen.

Ebenso wie Ammoniak lassen sich primäre oder sekundäre Amine als Aminierungsmittel verwenden.

Bevorzugt werden diese Aminierungsmittel zur Herstellung unsymmetrisch substituierter Di- oder Trialkylamine, wie z. B. Ethyldiisopropylamin und Ethyldicyclohexylamin, verwendet. Bevorzugt werden beispielsweise die folgenden Mono- und Dialkylamine als Aminierungsmittel verwendet: Methylamin, Dimethylamin, Ethylamin, Diethylamin, Propylamin, Diisopropylamin, Butylamin, Pentylamin, Hexylamin und Cyclohexylamin.

Das Aminierungsmittel kann bezüglich der aminierend zu hydrierenden Carbonylgruppe in stöchiometrischer Menge eingesetzt werden. Bevorzugt wird jedoch mit einem Überschuß an Aminierungsmittel gearbeitet und zwar im allgemeinen mit einem mehr als 1,05 molaren Überschuß pro Mol aminierend zu hydrierender Carbonylgruppe. Speziell Ammoniak wird im allgemeinen mit einem 1,05 bis 250-fachen, bevorzugt 2 bis 100-fachen, insbesondere 2 bis 50-fachen molaren Überschuß pro Mol umzusetzender Carbonylgruppe eingesetzt. Höhere Überschüsse sowohl an Ammoniak als auch an primären oder sekundären Aminen sind möglich.

Das erfindungsgemäße Verfahren läßt sich diskontinuierlich oder bevorzugt kontinuierlich wie folgt durchführen, wobei der Katalysator bevorzugt als Festbett im Reaktor angeordnet ist.

Die Aminierung der Aldehydgruppen oder Ketogruppen des Edukts kann in der Flüssigphase oder in der Gasphase durchgeführt werden.

Üblicherweise arbeitet man bei der Umsetzung bei Temperaturen von 50 bis 250°C, bevorzugt 50 bis 200°C, insbesondere 60 bis 170°C.

Im allgemeinen wird die Reaktion bei einem Druck von 1 bis 400 bar (0,1 bis 40 MPa) ausgeführt. Bevorzugt werden Drücke von 10 bis 250 bar, insbesondere von 20 bis 200 bar, angewandt.

Die Anwendung höherer Temperaturen und eines höheren Gesamtdrukkes ist möglich. Der Gesamtdruck im Reaktionsgefäß, welches sich aus der Summe der Partialdrücke des Aminierungsmittels, der Carbonylkomponente, der gebildeten Reaktionsprodukte sowie des gegebenenfalls mitverwendeten Lösungsmittels bei den angegebenen Temperaturen ergibt, wird zweckmäßigerweise durch Aufpressen von Wasserstoff auf den gewünschten Reaktionsdruck eingestellt.

Der Wasserstoff wird der Reaktion im allgemeinen in einer Menge von 5 bis 400 N1, bevorzugt in einer Menge von 50 bis 200 N1 pro Mol Carbonylkomponente zugeführt, wobei die Literangaben jeweils auf Normalbedingungen umgerechnet wurden (S.T.P.).

Die Umsetzung erfolgt im allgemeinen ohne zusätzliches Lösungsmittel. Bei der Umsetzung hochmolekularer hochviskoser oder bei Raumtemperatur fester Ausgangsverbindungen oder der Umsetzung zu entsprechenden Produkten kann es vorteilhaft sein, ein unter den Reaktionsbedingungen inertes Lösungsmittel, wie Methanol, Ethanol, Propanol, Tetrahydrofuran, Dioxan, N-Methylpyrrolidon, Mihagol oder Ethylenglykoldimethylether, mitzuverwenden.

Es kann für die Selektivität des vorliegenden Verfahrens vorteilhaft sein, die Katalysatorformkörper im Reaktor mit inerten Füllkörpern zu vermischen, sie sozusagen zu "verdünnen". Der Anteil der Füllkörper in solchen Katalysatorzubereitungen kann 20 bis 80, besonders 30 bis 60 und insbesonders 40 bis 50 Volumenteile betragen.

Praktisch geht man bei der Durchführung im allgemeinen so vor, dass man dem Katalysator, der sich üblicherweise in einem bevorzugt von außen beheizten Festbettreaktor befindet, bei der gewünschten Reaktionstemperatur und dem gewünschten Druck den Aldehyd oder das Keton und das Aminierungsmittel simultan zuführt. Dabei belastet man den Katalysator im allgemeinen mit 0,01 bis 5, bevorzugt 0,05 bis 3, und besonders bevorzugt mit 0,1 bis 1,6 1 Aldehyd oder Keton pro Liter Katalysator und Stunde. Hierbei ist es zweckmäßig, die Reaktanten bereits vor der Zuführung in das Reaktionsgefäß zu erwärmen.

Der Reaktor kann sowohl in der Sumpf- als auch in der Rieselfahrweise betrieben werden, d. h. man kann die Reaktanten sowohl von unten nach oben als auch von oben nach unten durch den Reaktor leiten. Es versteht sich von selbst, dass sich das Verfahren sowohl diskontinuierlich als auch kontinuierlich durchführen läßt. In beiden Fällen kann das überschüssige Aminierungsmittel zusammen mit dem Wasserstoff im Kreis geführt werden. Ist der Umsatz bei der Reaktion nicht vollständig, so kann das nicht umgesetzte Ausgangsmaterial ebenfalls in die Reaktionszone zurückgeführt werden.

Aus dem Reaktionsaustrag werden, nachdem dieser zweckmäßigerweise entspannt worden ist, das überschüssige Aminierungsmittel und der Wasserstoff entfernt und die erhaltenen aminierten Produkte durch Destillation, Flüssigextraktion oder Kristallisation gereinigt. Das überschüssige Aminierungsmittel und der Wasserstoff werden vorteilhaft wieder in die Reaktionszone zurückgeführt. Das gleiche gilt für die eventuell nicht oder nicht vollständig umgesetzte Carbonylkomponente oder eine entsprechende durch Hydrierung entstandene Alkoholkomponente.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der destillativen Aufarbeitung des Reaktionsproduktes aus diesem entfernt.

Die durch das erfindungsgemäße Verfahren erhältlichen Amine eignen sich u. a. als Zwischenprodukte bei der Herstellung von Kraftstoffadditiven (US-A-3 275 554; DE-A-21 25 039 und DE-A-36 11 230), Tensiden, Arznei- und Pflanzenschutzmitteln sowie von Vulkanisationsbeschleunigern.

### Beispiele

### A) Herstellung von Katalysator A (erfindungsgemäß)

Eine wässrige Lösung aus Nickelnitrat, Kupfernitrat, Kobaltnitrat und Zirkoniumacetat, die 2,39 Gew.-% NiO, 2,39 Gew.-% CoO, 0,94 Gew.-% CuO und 2,82 Gew.-% ZrO₂ enthielt, wurde gleichzeitig in einem Rührgefäß in einem konstanten Strom mit einer 20 Gew.-%igen wässrigen Natriumcarbonatlösung bei einer Temperatur von 70 °C, so gefällt, dass der mit einer Glaselektrode gemessene pH-Wert von 7,0 aufrechterhalten wurde.

Die erhaltene Suspension wurde filtriert und der Filterkuchen mit vollentsalztem Wasser gewaschen bis die elektrische Leitfähigkeit des Filtrats ca. 20 mS betrug. Danach wurde der Filterkuchen bei einer Temperatur von 150 °C in einem Trockenschrank oder einem Sprühtrockner getrocknet. Das auf diese Weise erhaltene Hydroxidcarbonatgemisch wurde nun bei einer Temperatur von 500 °C über einen Zeitraum von 4 Stunden getempert.

Der so erhaltene Katalysator A hatte die Zusammensetzung:
28 Gew.-% NiO, 28 Gew.-% CoO, 11 Gew.-% CuO, und 33 Gew.-% ZrO₂.

Das Katalysatorpulver wurde mit 3 Gew.-% Graphit vermischt und zu 5 x 3 mm-Tabletten verformt.

### B) Herstellung von Katalysator B (gemäß EP-A-382 049, Seite 6; für Vergleichsversuch)

Eine wässrige Lösung aus Zirkonium-, Kupfer(II)-, Cobalt(II)- und Nickel-(II)-salzen wurde simultan mit wässriger Natriumcarbonatlösung einer Dichte von 1,208 kg/l in eine Fällungsapparatur gepumpt, in der sich frisch gefälltes, in Wasser suspendiertes Zirkoniumdioxid befand. Der pH-Wert der Lösung wurde während der Fällung auf 6,0 konstant gehalten und nach Verbrauch der Metallsalzlösung durch Zugabe von wässriger Natriumcarbonatlösung auf pH 7,5 angehoben. Der Niederschlag wurde gewaschen, bei 120 °C bis zur Gewichtskonstanz getrocknet und bei 400 °C bis zur Gewichtskonstanz calciniert. Die erhaltene Katalysatormasse wurde vermahlen, mit 3 Gew.-% Graphit vermischt, tablettiert und nochmals bei 520 °C 3 Stunden calciniert.

Der so erhaltene Katalysator B hatte die Zusammensetzung:
76 Gew.-% ZrO₂, 4 Gew.-% CuO, 10 Gew.-% CoO und 10 Gew.-% NiO.

### C) Mechanische Stabilität der erfindungsgemäßen Katalysatoren

Die mechanische Stabilität von Katalysatoren mit 11 Gew.-% CuO, 33 Gew.-% ZrO₂ und insgesamt 56 Gew.-% [NiO + CoO] mit unterschiedlichen NiO/CoO-Verhältnissen, hergestellt wie für Katalysator A) beschrieben, wurde ermittelt.

Hierzu wurden die Katalysatoren unter Reaktionsbedingungen für 16 h wie folgt behandelt (= Kochtest) und danach auf ihre mechanischen Eigenschaften durch Ermittlung ihrer Seitendruckfestigkeit geprüft.

Dabei wurde so vorgegangen, dass ein Autoklav mit 30 g Katalysator (in einem Korb), 67 ml Isopropylamin, 67 ml Aceton und 16 ml Wasser befüllt wurde. Nach Verschließen des Autoklaven wurde der Autoklav mit Argon gespült. Der Autoklaveninhalt wurde während der Reaktionszeit mit 700 Umdrehungen/Min. gerührt. Anschließend wurden 50 bar H₂ aufgepresst und der Reaktorinhalt innerhalb von 120 Minuten auf 130 °C gebracht. Der Druck wurde mit H₂ bis auf 200 bar erhöht und 16 h bei der jeweiligen Temperatur gerührt. Nach Abkühlen des Autoklaven wurde sehr langsam entspannt, um ein Sprengen der Katalysatorformkörper durch sich bei der Entspannung ausdehnende gasförmige Reaktanden im Formkörper zu verhindern.

Nach Ausbau wurden die Tabletten auf ihre Härte durch Messung der Seitendruckfestigkeit überprüft.

### Ergebnisse:

| CoO-Gehalt des Katalysators in Gew.-%, wobei [NiO + CoO] = 56 Gew.-% | CoO-Gehalt bezogen auf den [NiO + CoO] -Gesamtgehalt in Gew.-% | Seitendruckfestigkeit (in N) nach Durchführung des Kochtests |
|---|---|---|
| 0 | 0,0 | 4 |
| 5 | 8,9 | 9 |
| 12 | 21,4 | 13 |
| 28 | 50,0 | 27 |

Die Seitendruckfestigkeit der Katalysatorformkörper steigt mit zunehmendem CoO/NiO-Verhältnis an.

Für das erfindungsgemäße Verfahren sind Katalysatoren bevorzugt, die nach dem oben definierten Kochtest eine Seitendruckfestigkeit von mindestens 5 Newton, insbesondere von mindestens 10 Newton, ganz besonders von mindestens 20 Newton, aufweisen.

Die Seitendruckfestigkeit wurde wie folgt bestimmt:

Die Katalysatortablette wurde zwischen zwei parallelen Platten auf der Mantelseite mit zunehmender Kraft belastet, bis Bruch eintrat. Die beim Bruch registrierte Kraft ist die Seitendruckfestigkeit. Die Bestimmung erfolgte auf einem Prüfgerät der Firma Zwick, Ulm, mit festsitzendem Drehteller und frei beweglichem, vertikalem Stempel, der den Formkörper gegen den festsitzenden Drehteller drückte. Der frei bewegliche Stempel war mit einer Druckmessdose zur Aufnahme der Kraft verbunden. Das Gerät wurde durch einen Computer gesteuert, welcher die Meßwerte registrierte und auswertete. Aus einer gut durchmischten Katalysatorprobe wurden 25 einwandfreie (d.h. rissefrei und ohne abgestoßenen Kanten) Tabletten entnommen, deren Seitendruckfestigkeiten einzeln ermittelt und anschließend gemittelt.

### Beispiel 1:

### Kontinuierliche Aminierung von Cyclopentanon

Ein kontinuierlich betriebener Hochdruckreaktor wurde mit 500 cm³ Katalysator A gefüllt und stündlich in Sumpffahrweise mit 300 cm³ Cyclopentanon und 750 g flüssigem Ammoniak beschickt. Die Katalysatortemperatur wurde auf 150 °C und der Druck im Reaktor, durch gleichzeitiges Aufpressen von Wasserstoff, auf 200 bar eingestellt. Aus dem Reaktionsaustrag wurde nach dessen Entspannung überschüssiges Ammoniak abdestilliert. Die gesammelten Reaktionsausträge wurden gaschromatographisch analysiert: Cyclopentylamin wurde in einer Ausbeute von 98,1% erhalten.

### Beispiel 2:

### Diskontinuierliche Aminierung von α-Tetralon

Ein 300 ml-Autoklav mit Magnetrührer und Katalysatorkorb wurde mit 30 ml α-Tetralon und 30 ml Katalyator A befüllt. Anschließend wurden 70 ml Ammoniak aufgepresst. Unter Rühren wurde mit Wasserstoff auf 100 bar nachgepresst, auf 100 °C erwärmt und mit Wasserstoff auf 200 bar nachgepresst. Es wurde 12 h bei 100 °C gerührt. Der Reaktionsaustrag wurde gaschromatographisch vermessen (Säule Rtx-5- Amin). Das Amin 1,2,3,4-Tetrahydro-1-naphthylamin wurde in einer Ausbeute von 97,9 % erhalten.

### Beispiel 3:

Kontinuierliche Aminierung von p-Methoxybenzaldehyd (Anisaldehyd) zu p-Methoxybenzylamin

Eine kontinuierliche betriebene Laborapparatur (gerader Durchgang, 100 ml Katalysatorvolumen) wurde mit Katalysator A befüllt. Anschliessend wurde Ammoniak kalt aufgepreßt (30 bar) und der Reaktor auf 130 °C erwärmt. Mit Wasserstoff wurde ein Druck von 200 bar eingestellt.
a) 65,5 g/h Anisaldehyd und 53 g/h Ammoniak wurden mit 20 Nl/h Wasserstoff bei 130°C von unten nach oben über den Kontakt gefahren: Ausbeute 98,6 %.
b) 67,5 g/h Anisaldehyd und 53 g/h Ammoniak wurden mit 20 Nl/h Wasserstoff bei 120°C von unten nach oben über den Kontakt gefahren: Ausbeute 99,2 %.
c) 32,7 g/h Anisaldehyd und 26 g/h Ammoniak wurden mit 20 Nl/h Wasserstoff bei 130°C von unten nach oben über den Kontakt gefahren: Ausbeute 98,5 %.

### Beispiel 4 (Vergleichsbeispiel):

Beispiel Nr. 1 zur kontinuierlichen Aminierung von Cyclopentanon wurde wie beschrieben durchgeführt, mit dem Unterschied, dass der Katalysator B eingesetzt wurde.

Der Katalysator war nach einer Versuchsdauer von wenigen Tagen (< 10) zerfallen, wies also eine ungenügende mechanische Stabilität auf, und war deswegen nicht geeignet.

### Beispiel 5:

### Kontinuierliche Aminierung von α-Indanon

Ein kontinuierlich betriebener Hochdruckreaktor wurde mit 250 cm³ Katalysator A gefüllt und bei 130°C und 100 bar stündlich in Sumpffahrweise mit 32 g einer methanolischen α-Indanon-Lösung (80 Gew.-% Indanon), 55 ml flüssigem Ammoniak und 15 Nl Wasserstoff beschickt. Überschüssiger Ammoniak und Methanol wurden anschließend abdestilliert. Die gesammelten Reaktionsausträge wurden gaschromatographisch analysiert: 1-Aminoindan wurde in einer Ausbeute von 91,8 % erhalten.

## Patentansprüche

1. Verfahren zur Herstellung von Aminen durch Umsetzung von Aldehyden oder Ketonen bei Temperaturen von 50 bis 250°C und Drücken von 0,1 bis 40 MPa mit Stickstoffverbindungen, ausgewählt aus der Gruppe Ammoniak, primäre und sekundäre Amine, und mit Wasserstoff in Gegenwart eines Katalysators, **dadurch gekennzeichnet, dass** die katalytisch aktive Masse des Katalysators nach dessen Herstellung und vor der Behandlung mit Wasserstoff
22 bis 45 Gew.-% sauerstoffhaltige Verbindungen des Zirkoniums, berechnet als ZrO₂,
1 bis 30 Gew.-% sauerstoffhaltige Verbindungen des Kupfers, berechnet als CuO,
5 bis 50 Gew.-% sauerstoffhaltige Verbindungen des Nickels, berechnet als NiO, wobei das Molverhältnis von Nickel zu Kupfer größer 1 ist,
5 bis 50 Gew.-% sauerstoffhaltige Verbindungen des Kobalts, berechnet als CoO,
0 bis 5 Gew.-% sauerstoffhaltige Verbindungen des Molybdäns, berechnet als MoO₃,
und 0 bis 10 Gew.-% sauerstoffhaltige Verbindungen des Aluminiums und/oder Mangans, berechnet als Al₂O₃ bzw. MnO₂, enthält.

2. Verfahren nach Anspruch 1, **dadurch gekennzeichnet, dass** die katalytisch aktive Masse des Katalysators nach dessen Herstellung und vor der Behandlung mit Wasserstoff
22 bis 39 Gew.-% sauerstoffhaltige Verbindungen des Zirkoniums, berechnet als ZrO₂,
2 bis 25 Gew.-% sauerstoffhaltige Verbindungen des Kupfers, berechnet als CuO,
15 bis 45 Gew.-% sauerstoffhaltige Verbindungen des Nickels, berechnet als NiO, wobei das Molverhältnis Nickel zu Kupfer größer 1 ist,
20 bis 45 Gew.-% sauerstoffhaltige Verbindungen des Kobalts, berechnet als CoO,
0 bis 5 Gew.-% sauerstoffhaltige Verbindungen des Molybdäns, berechnet als MoO₃,
und 0 bis 10 Gew.-% sauerstoffhaltige Verbindungen des Aluminiums und/oder Mangans, berechnet als Al₂O₃ bzw. MnO₂, enthält.

3. Verfahren nach den Ansprüchen 1 oder 2, **dadurch gekennzeichnet, dass** die katalytisch aktive Masse des Katalysators nach dessen Herstellung und vor der Behandlung mit Wasserstoff 25 bis 39 Gew.-% sauerstoffhaltige Verbindungen des Zirkoniums, berechnet als ZrO₂,enthält.

4. Verfahren nach einem der Ansprüche 1 bis 3, **dadurch gekennzeichnet, dass** die katalytisch aktive Masse des Katalysators nach dessen Herstellung und vor der Behandlung mit Wasserstoff 5 bis 15 Gew.-% sauerstoffhaltige Verbindungen des Kupfers, berechnet als CuO, enthält.

5. Verfahren nach einem der Ansprüche 1 bis 4, **dadurch gekennzeichnet, dass** die katalytisch aktive Masse des Katalysators nach dessen Herstellung und vor der Behandlung mit Wasserstoff in Summe 35 bis 69 Gew.-% sauerstoffhaltige Verbindungen des Nickels, berechnet als NiO, und sauerstoffhaltige Verbindungen des Kobalts, berechnet als CoO, enthält, wobei der Gehalt an sauerstoffhaltigen Verbindungen des Kobalts, berechnet als CoO, bezogen auf die Summe an sauerstoffhaltigen Verbindungen des Nickels, berechnet als NiO, und sauerstoffhaltigen Verbindungen des Kobalts, berechnet als CoO, mindestens 12,0 Gew.-%, insbesondere mindestens 40 Gew.-%, beträgt.

6. Verfahren nach einem der Ansprüche 1 bis 5, **dadurch gekennzeichnet, dass** die katalytisch aktive Masse des Katalysators nach dessen Herstellung und vor der Behandlung mit Wasserstoff 0,1 bis 5 Gew.-% sauerstoffhaltige Verbindungen des Molybdäns, berechnet als MoO₃, enthält.

7. Verfahren nach einem der Ansprüche 1 bis 6, **dadurch gekennzeichnet, dass** man den Katalysator in Form von Formkörpern einsetzt.

8. Verfahren nach einem der Ansprüche 1 bis 7 zur Herstellung von Aminen der Formel I 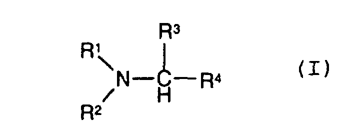 in der
R¹, R²
Wasserstoff, C₁₋₂₀-Alkyl, C₃₋₁₂-Cycloalkyl, Aryl, C₇₋₂₀-Aralkyl und C₇₋₂₀-Alkylaryl oder gemeinsam (CH₂)ⱼ-X-(CH₂)ₖ,
R³, R⁴
Wasserstoff, Alkyl, Cycloalkyl, Hydroxyalkyl, Aminoalkyl, Alkanolaminoalkyl, Alkoxyalkyl, Dialkylaminoalkyl, Alkylaminoalkyl, R⁵-(OCR⁶R⁷CR⁸R⁹)ₙ-(OCR⁶R⁷), Aryl, Heteroaryl, Aralkyl, Heteroarylalkyl, Alkylaryl, Alkylheteroaryl und
Y-(CH₂)ₘ-NR⁵-(CH₂)_{q} oder gemeinsam (CH₂)ₗ-X-(CH₂)ₘ oder
R² und R⁴
gemeinsam (CH₂)ₗ-X-(CH₂)ₘ,
R⁵, R¹⁰
Wasserstoff, C₁₋₄-Alkyl, C₇₋₄₀-Alkylphenyl,
R⁶, R⁷, R⁸, R⁹
Wasserstoff, Methyl oder Ethyl,
X
CH₂, CHR⁵, Sauerstoff, Schwefel oder NR⁵,
Y
N(R¹⁰)₂, Hydroxy, C₂₋₂₀-Alkylaminoalkyl oder C₃₋₂₀-Dialkylaminoalkyl,
n
eine ganze Zahl von 1 bis 30 und
j, k, l, m, q
eine ganze Zahl von 1 bis 4
bedeuten, durch Umsetzung von Aldehyden oder Ketonen der Formel II bzw. III 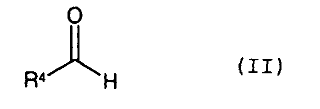 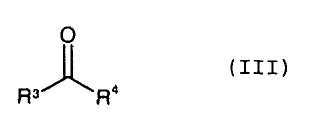 mit Stickstoffverbindungen der Formel IV

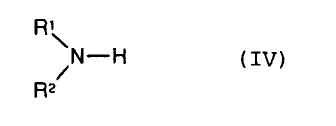

## Claims

1. A process for preparing amines by reacting aldehydes or ketones at temperatures of 50°C to 250°C and pressures of 0.1 to 40 MPa with nitrogen compounds selected from the group of ammonia, primary and secondary amines, and with hydrogen in the presence of a catalyst, wherein the catalytically active mass of the catalyst contains, after its preparation and before the treatment with hydrogen,
22 to 45% by weight of oxygen-containing compounds of zirconium, calculated as ZrO₂,
1 to 30% by weight of oxygen-containing compounds of copper, calculated as CuO,
5 to 50% by weight of oxygen-containing compounds of nickel, calculated as NiO, where the molar ratio of nickel to copper is greater than 1,
5 to 50% by weight of oxygen-containing compounds of cobalt, calculated as CoO,
0 to 5% by weight of oxygen-containing compounds of molybdenum, calculated as MoO₃,
and 0 to 10% by weight of oxygen-containing compounds of aluminum and/or manganese, calculated as Al₂O₃ or MnO₂.

2. A process as claimed in claim 1, wherein the catalytically active mass of the catalyst contains, after its preparation and before the treatment with hydrogen,
22 to 39% by weight of oxygen-containing compounds of zirconium, calculated as ZrO₂,
2 to 25% by weight of oxygen-containing compounds of copper, calculated as CuO,
15 to 45% by weight of oxygen-containing compounds of nickel, calculated as NiO, where the molar ratio of nickel to copper is greater than 1,
20 to 45% by weight of oxygen-containing compounds of cobalt, calculated as CoO,
0 to 5% by weight of oxygen-containing compounds of molybdenum, calculated as MoO₃,
and 0 to 10% by weight of oxygen-containing compounds of aluminum and/or manganese, calculated as Al₂O₃ or MnO₂.

3. A process as claimed in claims 1 or 2, wherein the catalytically active mass of the catalyst contains, after its preparation and before the treatment with hydrogen, 25 to 39% by weight of oxygen-containing compounds of zirconium, calculated as ZrO₂.

4. A process as claimed in any of claims 1 to 3, wherein the catalytically active mass of the catalyst contains, after its preparation and before the treatment with hydrogen, 5 to 15% by weight of oxygen-containing compounds of copper, calculated as CuO.

5. A process as claimed in any of claims 1 to 4, wherein the catalytically active mass of the catalyst contains, after its preparation and before the treatment with hydrogen, in total 35 to 69% by weight of oxygen-containing compounds of nickel, calculated as NiO, and oxygen-containing compounds of cobalt, calculated as CoO, where the content of oxygen-containing compounds of cobalt, calculated as CoO, based on the total of oxygen-containing compounds of nickel, calculated as NiO, and oxygen-containing compounds of cobalt, calculated as CoO, is at least 12.0% by weight, in particular at least 40% by weight.

6. A process as claimed in any of claims 1 to 5, wherein the catalytically active mass of the catalyst contains, after its preparation and before the treatment with hydrogen, 0.1 to 5% by weight of oxygen-containing compounds of molybdenum, calculated as MoO₃.

7. A process as claimed in any of claims 1 to 6, wherein the catalyst is employed in the form of shaped articles.

8. A process as claimed in any of claims 1 to 7 for preparing amines of the formula I 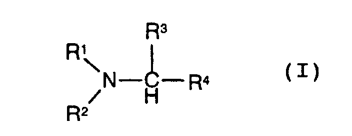 in which
R¹, R²
are hydrogen, C₁₋₂₀-alkyl, C₃₋₁₂-cycloalkyl, aryl, C₇₋₂₀-aralkyl and C₇₋₂₀-alkylaryl or together are (CH₂)ⱼ-X-(CH₂)ₖ,
R³, R⁴
are hydrogen, alkyl, cycloalkyl, hydroxyalkyl, aminoalkyl, alkanolaminoalkyl, alkoxyalkyl, dialkylaminoalkyl, alkylaminoalkyl, R⁵-(OCR⁶R⁷CR⁸R⁹)ₙ-(OCR⁶R⁷), aryl, hetaryl, aralkyl, hetarylalkyl, alkylaryl, alkylhetaryl and
Y-(CH₂)ₘ-NR⁵-(CH₂)_{q} or together (CH₂)ₗ-X-(CH₂)ₘ or
R² and R⁴
are together (CH₂)ₗ-X-(CH₂)ₘ,
R⁵, R¹⁰
are hydrogen, C₁₋₄-alkyl, C₇₋₄₀-alkylphenyl,
R⁶, R⁷, R⁸, R⁹
are hydrogen, methyl or ethyl,
X
is CH₂, CHR⁵, oxygen, sulfur or NR⁵,
Y
is N(R¹⁰)₂, hydroxyl, C₂₋₂₀-alkylaminoalkyl or C₃₋₂₀-dialkylaminoalkyl,
n
is an integer from 1 to 30 and
j, k, l, m, q are an integer from 1 to 4
by reacting aldehydes or ketones of the formula II or III 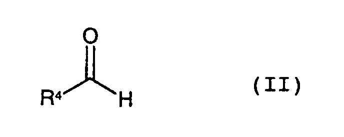 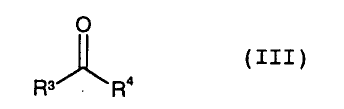 with nitrogen compounds of the formula IV

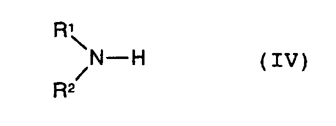

## Revendications

1. Procédé de préparation d'amines au moyen de la réaction d'aldéhydes ou de cétones à des températures allant de 50°C à 250°C et sous des pressions allant de 0,1 MPa à 40 MPa avec des composés azotés, choisis dans le groupe formé par l'ammoniac, les amines primaires et secondaires, et avec de l'hydrogène en présence d'un catalyseur, **caractérisé en ce que** la masse à activité catalytique du catalyseur contient, après sa préparation et avant le traitement avec l'hydrogène,
22% à 45% en poids de composés oxygénés du zirconium, exprimé en ZrO₂,
1% à 30% en poids de composés oxygénés du cuivre, exprimé en CuO,
5% à 50% en poids de composés oxygénés du nickel, exprimé en NiO, le rapport molaire du nickel au cuivre étant supérieur à 1,
5% à 50% en poids de composés oxygénés du cobalt, exprimé en CoO,
0% à 5% en poids de composés oxygénés du molybdène, exprimé en MoO₃,
et 0% à 10% en poids de composés oxygénés de l'aluminium et/ou du manganèse, exprimés en Al₂O₃ ou MnO₂.

2. Procédé selon la revendication 1, **caractérisé en ce que** la masse à activité catalytique du catalyseur contient, après sa préparation et avant le traitement avec l'hydrogène,
22% à 39% en poids de composés oxygénés du zirconium, exprimé en ZrO₂,
2% à 25% en poids de composés oxygénés du cuivre, exprimé en CuO,
15% à 45% en poids de composés oxygénés du nickel, exprimé en NiO, le rapport molaire du nickel au cuivre étant supérieur à 1,
20% à 45% en poids de composés oxygénés du cobalt, exprimé en CoO,
0% à 5% en poids de composés oxygénés du molybdène, exprimé en MoO₃,
et 0% à 10% en poids de composés oxygénés de l'aluminium et/ou du manganèse, exprimés en Al₂O₃ ou MnO₂.

3. Procédé selon la revendication 1 ou 2, **caractérisé en ce que** la masse à activité catalytique du catalyseur contient, après sa préparation et avant le traitement avec l'hydrogène, 25% à 39% en poids de composés oxygénés du zirconium, exprimé en ZrO₂.

4. Procédé selon l'une quelconque des revendications 1 à 3, **caractérisé en ce que** la masse à activité catalytique du catalyseur contient, après sa préparation et avant le traitement avec l'hydrogène, 5% à 15% en poids de composés oxygénés du cuivre, exprimé en CuO.

5. Procédé selon l'une quelconque des revendications 1 à 4, **caractérisé en ce que** la masse à activité catalytique du catalyseur contient, après sa préparation et avant le traitement avec l'hydrogène, au total 35% à 69% en poids de composés oxygénés du nickel, exprimé en NiO, et de composés oxygénés du cobalt, exprimé en CoO, dans lequel la teneur en composés oxygénés du cobalt, exprimé en CoO, par rapport au total des composés oxygénés du nickel, exprimé en NiO, et des composés oxygénés du cobalt, exprimé en CoO, représente au moins 12,0% en poids, en particulier au moins 40% en poids.

6. Procédé selon l'une quelconque des revendications 1 à 5, **caractérisé en ce que** la masse à activité catalytique du catalyseur contient, après sa préparation et avant le traitement avec l'hydrogène, 0,1% à 5% en poids de composés oxygénés du molybdène, exprimé en MoO₃.

7. Procédé selon l'une quelconque des revendications 1 à 6, **caractérisé en ce que** l'on met en oeuvre le catalyseur sous forme de corps moulés.

8. Procédé selon l'une quelconque des revendications 1 à 7 pour la préparation d'amines de formule I 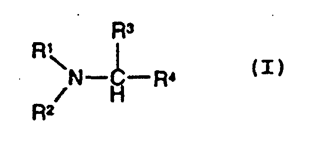 dans laquelle
R¹, R²
représentent un atome d'hydrogène, un groupe alkyle en C₁ à C₂₀, cycloalkyle en C₃ à C₁₂, aryle, aralkyle en C₇ à C₂₀ et alkylaryle en C₇ à C₂₀, ou représentent ensemble (CH₂)ⱼ-X-(CH₂)ₖ,
R³, R⁴ représentent un atome d'hydrogène, un groupe alkyle, cycloalkyle, hydroxyalkyle, aminoalkyle, alcanolaminoalkyle, alcoxyalkyle, dialkylaminoalkyle, alkylaminoalkyle, R⁵-(OCR⁶R⁷CR⁸R⁹)ₙ-(OCR⁶R⁷), aryle, hétéroaryle, aralkyle, hétéroarylalkyle, alkylaryle, alkylhétéroaryle et Y-(CH₂)ₘ-NR⁵-(CH₂)_{q}, ou représentent ensemble (CH₂)ₗ-X-(CH₂)ₘ, ou bien
R² et R⁴
représentent ensemble (CH₂)ₗ-X-(CH₂)ₘ,
R⁵, R¹⁰
représentent un atome d'hydrogène, un groupe alkyle en C₁ à C₄, alkylphényle en C₇ à C₄₀,
R⁶, R⁷, R⁸, R⁹
représentent un atome d'hydrogène, un groupe méthyle ou éthyle,
X
représente CH₂, CHR⁵, un atome d'oxygène, de soufre ou NR⁵,
Y
représente N(R¹⁰)₂, un groupe hydroxy, alkylaminoalkyle en C₂ à C₂₀ ou dialkylaminoalkyle en C₃ à C₂₀,
n
représente un nombre entier de 1 à 30 et
j, k, l, m, q
représentent un nombre entier de 1 à 4
au moyen de la réaction d'aldéhydes ou de cétones de formule II ou III 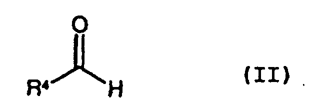 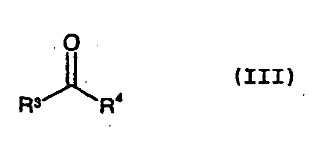 avec des composés azotés de formule IV p
